# EUROPÄISCHE PATENTSCHRIFT

(11) **EP 3 397 181 B1**
(45) Veröffentlichungstag und Bekanntmachung des Hinweises auf die Patenterteilung: **21.12.2022**
(21) Anmeldenummer: 16823020.9
(22) Anmeldetag: 30.12.2016
(51) Int. Cl.: A61B 18/12, A61B 18/14

(54) **ELEKTROCHIRURGIESYSTEM ZUM GENERIEREN VON HOCHFREQUENTEM WECHSELSTROM**
ELECTROSURGICAL SYSTEM FOR GENERATING HIGH-FREQUENCY ALTERNATING CURRENT
SYSTÈME ÉLECTROCHIRURGICAL POUR GÉNÉRER UN COURANT ALTERNATIF HAUTE FRÉQUENCE

(30) Priorität: 30.12.2015 DE 102015226846
(43) Veröffentlichungstag der Anmeldung: 07.11.2018
(73) Patentinhaber: Olympus Winter & Ibe GmbH, 22045 Hamburg (DE)
(72) Erfinder: WINTER, Hanno, 13353 Berlin (DE); KIRFE, Tino, 04617 Lödla (DE); BREITSPRECHER, Frank, 12527 Berlin (DE)
(74) Vertreter: Eisenführ Speiser
(86) Internationale Anmeldenummer: PCT/EP2016/082938
(87) Internationale Veröffentlichungsnummer: WO 2017/114957

(56) Entgegenhaltungen:
- EP-A1- 2 213 256
- DE-A1-102012 220 658
- US-A1- 2002 032 439
- US-A1- 2002 151 884
- US-A1- 2006 217 707
- US-A1- 2012 101 538

## Beschreibung

Die Erfindung betrifft ein Elektrochirurgiesystem zum Generieren von hochfrequentem Wechselstrom für eine Ablation von Körpergewebe.

Hochfrequenter Wechselstrom für die Ablation von Körpergewebe wird unter anderem für eine endovenöse Behandlung von Veneninsuffizienz genutzt. Dabei wird Hochfrequenzenergie lokal und präzise dosiert über einen Applikator auf zu behandelndes Venengewebe appliziert, was bewirkt, dass sich eine entsprechende Vene zusammenzieht und verschließt. Dies wird auch als Thermo-Okklusion bezeichnet. Gegenüber herkömmlichen Verfahren wie dem Venenstripping und auch im Vergleich zu anderen endovenösen Verfahren, die z.B. einen Laser nutzen, ist die Hochfrequenzablation eine besonders schonende Methode und darüber hinaus einfach und sicher in der Durchführung. Die Schmerzbelastung sowie das Risiko von Narben, Infektionen und Hämatomen sind ebenfalls sehr gering. Ein weiterer Vorteil der Hochfrequenzablation liegt darin, dass sie ambulant durchgeführt werden kann und die behandelten Patienten schon kurze Zeit nach einem entsprechenden Eingriff wieder mobil sind.

Die Hochfrequenzablation findet auch Verwendung zur Tumorbehandlung. DE102012220658 und US2002151884 zeigen Elektrochirurgiesysteme des Stands der Technik.

Der Erfindung liegt die Aufgabe zugrunde, ein verbessertes Elektrochirurgiesystem, insbesondere für eine Anwendung bei der Ablation von Körpergewebe, bereitzustellen.

Erfindungsgemäß wird zur Lösung dieser Aufgabe ein Elektrochirurgiesystem zum Generieren von hochfrequentem Wechselstrom für eine Ablation von Körpergewebe vorgeschlagen, das eine Hochfrequenz-Spannungsversorgungseinheit aufweist, die mit einer ersten und zweiten Elektrode elektrisch verbunden oder zu verbinden ist und die ausgebildet ist, diese Elektroden mit einer hochfrequenten Wechselspannung zu versorgen. Weiterhin hat das erfindungsgemäße Elektrochirurgiesystem eine Feedbackeinheit, die mit der ersten und zweiten Elektrode elektrisch verbunden ist und die ausgebildet ist, ein von einem elektrischen Widerstand zwischen der ersten und der zweiten Elektrode abhängiges Ausgabesignal zu generieren. Zum Steuern der Hochfrequenz-Spannungsversorgungsanlage weist das Elektrochirurgiesystem eine Steuereinheit auf, die ausgebildet ist, die Hochfrequenz-Spannungsversorgungseinheit derart zu steuern, dass zwischen mindestens einem ersten und einem zweiten Betriebsmodus automatisch wechseln kann, wobei im ersten Betriebsmodus die Wechselspannung durch einen ersten Grenzwert U₁ und im zweiten Betriebsmodus die Wechselspannung durch einen zweiten Grenzwert U₂ begrenzt wird, wobei U₁ größer ist als U₂. Ein automatischer Wechsel aus dem ersten Betriebsmodus in den zweiten Betriebsmodus erfolgt dann, wenn eine Anstiegsgeschwindigkeit des elektrischen Widerstands zwischen der ersten und zweiten Elektrode über einem vorbestimmten Grenzwert G liegt.

Die Erfindung schließt die Erkenntnis ein, dass bei der Koagulation von Gewebe der Widerstand des Gewebes von ausgehend einem geringen Gewebewiderstand hin zu einem hohen Widerstand eines ausgetrockneten Gewebes ansteigt. Der jeweilige vom Elektrochirurgiesystem erfasste Gewebewiderstand wird dem Nutzer durch ein wahrnehmbares Signal, z.B. ein akustisches Signal angezeigt. Das wahrnehmbare Signal ändert sich mit dem Gewebewiderstand z.B. kann die Frequenz eines akustischen Signals den erfassten Gewebewiderstand anzeigen, so dass die Tonhöhe des Signals mit steigendem Gewebewiderstand ansteigt. Bei der Venenablation kann der Nutzer, z.B. der behandelnde Arzt, die Geschwindigkeit, mit der er den Applikator entlang der Vene führt, je nach wahrgenommenem Tonsignal variieren. Ein Problem ist dabei, dass kurz vor einem Austrocknen des Gewebes und der damit verbundenen Gefahr eines Anhaftens des Applikators der Gewebewiderstand sehr schnell und praktisch sprunghaft ansteigt, so dass der Arzt häufig nicht schnell genug reagieren, d.h. den Applikator schnell genug weiterbewegen kann.

Mit dem erfindungsgemäßen Elektrochirurgiesystem kann ein quasi sprunghafter Anstieg des Gewebewiderstandes vermieden werden, indem schon zu Beginn dieses sprunghaften Anstiegs eine über die erste und zweite Elektrode abgegebene Leistung durch eine Verringerung der die Elektroden versorgenden Wechselspannung verringert wird. Daher ist der zweite Grenzwert U₂ bevorzugt so zu wählen, dass die Heizleistung durch einen Wechsel von dem ersten in den zweiten Betriebsmodus deutlich reduziert ist.

Durch eine solche automatische Verringerung der Heizleistung hat der Nutzer des Elektrochirurgiesystems mehr Zeit, um auf das Ausgabesignal und den dadurch implizierten elektrischen Widerstand des Körpergewebes zwischen den beiden Elektroden zu reagieren. Eine Reaktion kann dabei typischerweise in einem Verändern einer Position der ersten und zweiten Elektrode am zu behandelnden Körpergewebes bestehen.

Vorteilhaft an dem erfindungsgemäßen Elektrochirurgiesystem ist daher, dass eine Austrocknung von mit dem Elektrochirurgiesystem behandeltem Körpergewebe und damit ein Anhaften des Applikators vermieden werden kann. Dadurch kann beispielsweise eine Antrocknung von Blut an der ersten oder zweiten Elektrode verhindert werden. Weiterhin kann durch eine bessere Kontrolle über die Koagulation des Gewebes eine für die Behandlung notwendige Therapiezeit verkürzt werden, da Therapiepausen, beispielsweise zur Reinigung der beiden Elektroden von ausgetrocknetem Gewebe, vermieden werden.

Ein erfindungsgemäßes Elektrochirurgiesystem erlaubt es außerdem, anfänglich eine hohe Heizleistung in das Gewebe einzubringen, die zum schnellen Erwärmen von zu behandelndem Körpergewebe führt. Vor einem Austrocknen des Körpergewebes reduziert die Steuereinheit automatisch die Spannung und somit auch die Heizleistung. Daher kann in einer Therapiephase, in der eine schnelles Erhitzen des Körpergewebes erwünscht ist, eine sehr hohe Heizleistung des Elektrochirurgiegeräts angewendet werden ohne dass dadurch eine erhöhte Gefahr der Austrocknung des Gewebes existieren muss. Dies trägt ebenfalls zu einer kurzen Therapiezeit bei. Daher ist das erfindungsgemäße Elektrochirurgiegerät besonders sicher und einfach zu nutzen. Insbesondere eine schnelle Reaktion des Nutzers, um ein Austrocknen des Gewebes zu vermeiden, entfällt.

Der automatische Wechsel zwischen Betriebsmodi der Steuereinheit bedeutet nicht, dass ein zusätzlich möglicher manueller Wechsel ausgeschlossen ist. Vielmehr ist in einer Ausführungsvariante des Elektrochirurgiesystems die Steuereinheit weiterhin ausgebildet, ein Eingabesignal eines Nutzers zu empfangen, und einen Wechsel zwischen dem ersten und dem zweiten Betriebsmodus abhängig von dem Eingabesignal auszuführen. Nachfolgend werden bevorzugte Ausführungsvarianten des erfindungsgemäßen Elektrochirurgiesystems beschrieben.

Erfindungsgemäß ist die Steuereinheit weiterhin ausgebildet, die Hochfrequenz-Spannungsversorgungseinheit derart zu steuern, dass ein automatischer Wechsel aus dem zweiten Betriebsmodus in den ersten Betriebsmodus dann erfolgt, wenn der elektrische Widerstand zwischen der ersten und der zweiten Elektrode unter einem vorbestimmten Grenzwert liegt. Das Elektrochirurgiesystem kann somit nach einem automatisch Umschalten in einen Betriebsmodus mit geringer Heizleistung wieder automatisch zu einer hohen Heizleistung zurückschalten, falls das die beiden Elektroden umgebende Gewebe keinen zu hohen elektrischen Widerstand aufweist. Hierbei ist der vorbestimmte Grenzwert W bevorzugt so gewählt, dass ein Zurückschalten dann automatisch erfolgt, wenn dieses Gewebe nicht bereits ausgetrocknet ist. Hierdurch wird die Therapiezeit verkürzt, da der Nutzer des Elektrochirurgiesystems nicht erst manuell den elektrischen Widerstand des Gewebes überprüfen muss, um gegebenenfalls die an den beiden Elektroden anliegende Wechselspannung und somit die Heizleistung zu verstellen. Weiterhin ist vorteilhaft, dass zu Beginn einer Behandlung des Körpergewebes die Wechselspannung automatisch auf den ersten Grenzwert U₁ begrenzt wird, so dass die zu Beginn einer Behandlung notwendige hohe Heizleistung von dem erfindungsgemäßen Elektrochirurgiesystem bereitgestellt wird.

Vorzugsweise umfasst das Elektrochirurgiesystem weiterhin eine Verarbeitungseinheit, die mit der ersten und der zweiten Elektrode elektrisch verbunden ist und ausgebildet ist, über die erste und die zweite Elektrode ein elektrisches Antwortsignal zu empfangen, daraus den elektrischen Widerstand zwischen der der ersten und der zweiten Elektrode oder einen den elektrischen Widerstand repräsentierenden Wert zu bestimmen und abhängig von dem elektrischen Widerstand ein Steuersignal zu generieren und auszugeben, und wobei die Feedbackeinheit und die Steuereinheit weiterhin ausgebildet sind, das Steuersignal zu empfangen.

In einer besonders bevorzugten Ausführungsvariante weist die Feedbackeinheit einen Lautsprecher auf und ist ausgebildet, das Ausgabesignal über den Lautsprecher als hörbaren Ton auszugeben. Eine solche Ausgabe des Tons kann derart erfolgen, dass eine Frequenz des hörbaren Tons z.B. proportional zu dem elektrischen Widerstand zwischen der ersten und der Zweiten Elektrode ist. In einem weiteren Beispiel dieser Ausführungsvariante erfolgt die Ausgabe des Tons über eine Folge einzelner Töne mit jeweiligen Tonunterbrechungen. In diesem Beispiel ist eine Dauer der jeweiligen Tonunterbrechungen abhängig von dem elektrischen Widerstand zwischen der ersten und zweiten Elektrode. Die Feedbackeinheit kann auch so ausgebildet sein, dass eine Kombination aus Frequenzänderung und Tonunterbrechungsdauer als Ausgabesignal ausgegeben wird. Beispielsweise kann sich für eine zu große Anstiegsgeschwindigkeit des elektrischen Widerstands nicht nur die Frequenz des hörbaren Tons ändert, sondern es können einzelne Töne, insbesondere als Piep-Geräusch, ausgegeben werden. Die Ausgabe des Ausgabesignals als hörbaren Ton hat hierbei den Vorteil, dass ein Nutzer des Elektrochirurgiesystems nicht zusätzlich eine optische Ausgabe, wie beispielsweise einen Monitor, betrachten muss, um in Form des Ausgabesignals eine Information über den elektrischen Widerstand zwischen der ersten und zweiten Elektrode zu erhalten.

In einer alternativen Ausführungsvariante weist die Feedbackeinheit eine optische Ausgabe auf und ist ausgebildet, das Ausgabesignal über die optische Ausgabe als wahrnehmbare visuelle Information auszugeben. Eine solche visuelle Information kann beispielsweise in einer Veränderung einer Farbe von Bildpunkten der optischen Ausgabe liegen oder in einer Veränderung eines Betriebszustands einer Leuchtdiode.

In einer weiteren Ausführungsvariante ist die Feedbackeinheit ausgebildet, mit einer externen optischen Ausgabe oder mit einem externen Lautsprecher elektrisch leitend verbunden zu werden. In dieser Ausführungsvariante wird eine Reparatur des Lautsprechers oder der optischen Ausgabe vereinfacht, da das jeweilige externe Bauteil ausgewechselt werden kann, ohne das Elektrochirurgiesystem auswechseln oder reparieren zu müssen.

Vorzugsweise beträgt der zweite Grenzwert U₂ zwischen 50% und 80%, bevorzugt 70%, des ersten Grenzwerts U₁. Ein solches Verhältnis aus zweitem Grenzwert U₂ und erstem Grenzwert U₁ hat sich als besonders vorteilhaft herausgestellt, um ein zu behandelndes Körpergewebe vor dem Austrocknen zu bewahren. Bei 50% bis 80% des Grenzwerts U₁ ist die entsprechende Heizleistung der beiden Elektroden so weit verringert, dass die Anstiegsgeschwindigkeit des elektrischen Widerstands zwischen den beiden Elektroden derart reduziert ist, dass der Nutzer des Elektrochirurgiesystems ausreichend Zeit hat, um als Reaktion auf das Ausgabesignal eine Position der Elektroden am Gewebe zu verändern und dadurch eine Austrocknung des entsprechenden Körpergewebes zu verhindern.

In einer weiteren Ausführungsvariante kann die Amplitude der Wechselspannung U₁ und/oder die Amplitude der Wechselspannung U₂ manuell von dem Nutzer über eine Nutzer-Schnittstelle eingegeben und von der Steuereinheit als von der Nutzer-Schnittstelle bereitgestelltes Nutzersignal empfangen werden. Typische Werte für den Grenzwert U₁ liegen im Bereich von 80V bis 200V, beispielsweise 100V.

In einer besonders bevorzugten Ausführungsvariante ist die Steuereinheit weiterhin ausgebildet, den elektrischen Widerstand zwischen der ersten Elektrode und der zweiten Elektrode oder einen den Widerstand repräsentierenden Wert über einen vorbestimmten Zeitraum zu speichern, den elektrischen Widerstand oder den repräsentierenden Wert zu einem aktuellen Zeitpunkt mit dem elektrischen Widerstand oder dem repräsentierenden Wert zu einem früheren Zeitpunkt zu vergleichen und dadurch die Anstiegsgeschwindigkeit des elektrischen Widerstands zu bestimmen. Hierbei kann das Bestimmen der Anstiegsgeschwindigkeit auch über eine Vielzahl von zu einem früheren Zeitpunkt aufgenommenen elektrischen Widerständen oder den Widerstand repräsentierenden Werten erfolgen. Eine Erhöhung der Anstiegsgeschwindigkeit des elektrischen Widerstands kann auch dadurch bestimmt werden, dass nach festen Zeitschritten der elektrische Widerstand oder der den Widerstand repräsentierende Wert bestimmt wird, und wenn die Differenz zu dem vor dem Zeitschritt ermittelten Wert über einem vorbestimmten Grenzwert liegt, wird eine zu große Anstiegsgeschwindigkeit des elektrischen Widerstands detektiert und ein Wechsel vom ersten zum zweiten Betriebsmodus durch die Steuereinheit ausgeführt.

In einer Ausführungsvariante ist die Steuereinheit weiterhin ausgebildet, zwischen einer Anzahl von weiteren Betriebsmodi mit weiteren Grenzwerten U₃,..., Uₙ mit der Reihe nach absteigenden Amplituden, die kleiner als die Amplitude von U₂ sind, automatisch zu wechseln, wobei ein automatischer Wechsel aus einem der Betriebsmodi mit dem Grenzwert Uᵢ in den Betriebsmodus mit dem nächst kleineren Grenzwert Uᵢ₊₁ für die entsprechende Wechselspannung dann erfolgt, wenn die Anstiegsgeschwindigkeit des elektrischen Widerstands zwischen der ersten und der zweiten Elektrode über einem vorbestimmten und dem Betriebsmodus mit dem Grenzwert Uᵢ zugeordneten Grenzwert Gᵢ liegt. Hierdurch kann in vorteilhafter Weise die Heizleistung der beiden Elektroden weiter reduziert werden. Dies kann unter anderem dann sinnvoll sein, wenn der elektrische Widerstand des zu behandelnden Gewebes auch bei Vorliegen der im Vergleich zu der Wechselspannung mit dem Grenzwert U₁ reduzierten Wechselspannung mit dem Grenzwert U₂ eine große Anstiegsgeschwindigkeit aufweist. In diesem Fall sollte die Heizleistung der beiden Elektroden weiter reduziert werden, um ein zu starkes Austrocknen des Körpergewebes zu vermeiden.

Vorzugsweise weist das erfindungsgemäße Elektrochirurgiesystem einen Applikator auf, bei dem die erste und die zweite Elektrode in dem Applikator eine bipolare Elektrodenkonfiguration bilden, wobei die erste Elektrode an einer vorzugsweise abgerundeten Spitze eines distalen Endes des Applikators angeordnet ist und die zweite Elektrode zwischen 0,5 cm und 3 cm von der ersten Elektrode beabstandet an dem distalen Ende des Applikators angeordnet ist. In einer alternativen Ausführungsvariante sind die Elektroden auf zwei verschiedenen distalen Enden des Applikators angeordnet, wobei die beiden distalen Enden zueinander benachbart sind. Vorzugsweise ist der Applikator so ausgebildet, dass er über einen Katheter zu dem zu behandelnden Körpergewebe zuführbar ist. Dies erleichtert eine operative Behandlung des Körpergewebes und reduziert dabei die Narbenbildung und die Regenrationszeit eines Patienten nach einer solchen Behandlung, da nur wenig gesundes Körpergewebe des Patienten geschädigt werden muss.

Zur Lösung der vorgenannten Aufgabe wird außerdem ein Verfahren zum Betreiben eines Elektrochirurgiesystems vorgeschlagen, welches nicht Teil der Erfindung ist, das die folgenden Verfahrensschritte aufweist:
- Anlegen einer Wechselspannung an eine erste Elektrode und an eine zweite Elektrode eines Applikators,
- Generieren und Ausgeben eines Ausgabesignals, das von einem elektrischen Widerstand zwischen der ersten und der zweiten Elektrode abhängig ist,
- Steuern der Wechselspannung derart, dass zwischen mindestens einem ersten und einem zweiten Betriebsmodus des Applikators automatisiert gewechselt werden kann, wobei im ersten Betriebsmodus Wechselspannung durch einen ersten Grenzwert U₁ und im zweiten Betriebsmodus durch einen zweiten Grenzwert U₂ begrenzt ist, wobei U₁ größer ist als U₂ und wobei ein automatisierter Wechsel aus dem ersten Betriebsmodus in den zweiten Betriebsmodus dann erfolgt, wenn eine Anstiegsgeschwindigkeit des elektrischen Widerstands zwischen der ersten und zweiten Elektrode über einem vorbestimmten Grenzwert G liegt.

Verzugsweise erfolgt das Steuern der Wechselspannung derart, dass ein automatischer Wechsel aus dem zweiten Betriebsmodus in den ersten Betriebsmodus dann erfolgt, wenn der elektrische Widerstand zwischen der ersten und der zweiten Elektrode unter einem vorbestimmten Grenzwert W liegt.

In einer bevorzugten Ausführungsvariante weist das Verfahren die folgenden weiteren Verfahrensschritte auf:
- Bestimmen des elektrischen Widerstands oder eines den elektrischen Widerstand repräsentierenden Werts zwischen der ersten und der zweiten Elektrode,
- Generieren und Ausgeben eines Steuersignals, das von dem elektrischen Widerstand abhängig ist,
- Speichern des elektrischen Widerstands oder des den elektrischen Widerstand repräsentierenden Werts über einen vorbestimmten Zeitraum und Bestimmen der Anstiegsgeschwindigkeit des elektrischen Widerstands durch ein Vergleichen des elektrischen Widerstands oder des repräsentierenden Werts zu einem aktuellen Zeitpunkt mit dem elektrischen Widerstand oder dem repräsentierenden Wert zu einem früheren Zeitpunkt.

Ein weiterer Aspekt der Offenbarung, der nicht Teil der Erfindung ist, betrifft das Verwenden eines Elektrochirurgiesystems der vorbeschrieben Art zur Thermonekrose von unerwünschtem Körpergewebe, insbesondere zur endovenösen Behandlung von Veneninsuffizienz.

Ein weiterer Aspekt der Offenbarung, der nicht Teil der Erfindung ist, betrifft das Anwenden des vorbeschriebenen Verfahrens im Rahmen einer Ablation von unerwünschtem Gewebe, insbesondere bei einer endovenösen Behandlung von Veneninsuffizienz.

Die Erfindung soll nun anhand von Ausführungsbeispielen mit Bezug auf die Figuren näher erläutert werden. Von den Figuren illustriert:
- Fig. 1: ein Elektrochirurgiesystem zum Generieren von hochfrequentem Wechselstrom mit einem Applikator gemäß einem erfindungsgemäßen Ausführungsbeispiel;
- Fig. 2: einen typischen Verlauf des elektrischen Widerstands zwischen einer ersten und einer zweiten Elektrode über die Zeit bei der Thermonekrose von Körpergewebe;
- Fig. 3: einen Verlauf der Abhängigkeit zwischen einer Heizleistung bei einer Ablation von Körpergewebe und einem elektrischen Widerstand, wobei ein Einfluss einer Spannungsreduktion von U₁ auf U₂ veranschaulicht wird; und
- Fig. 4: ein erfindungsgemäßes Verfahren zum Betreiben eines Elektrochirurgiesystems gemäß einem weiteren Aspekt der Erfindung.

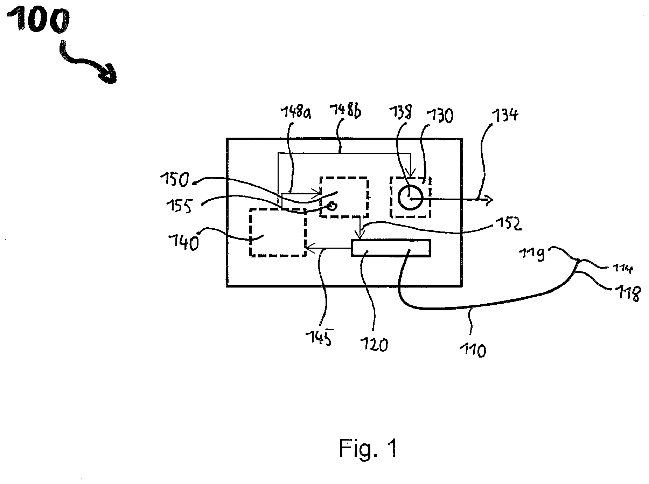

Fig. 1 zeigt ein Elektrochirurgiesystem 100 zum Generieren von hochfrequentem Wechselstrom mit einem Applikator 110 gemäß einem erfindungsgemäßen Ausführungsbeispiel.

Das Elektrochirurgiesystem 100 umfasst den Applikator 110, eine Hochfrequenz-Spannungsversorgungseinheit 120, eine Feedbackeinheit 130, eine Verarbeitungseinheit 140 und eine Steuereinheit 150.

Die Hochfrequenz-Spannungsversorgungseinheit 120 ist mit einer ersten und zweiten Elektrode 114, 118 elektrisch verbunden und ausgebildet, diese Elektroden 114, 118 mit einer Wechselspannung zu versorgen. Die beiden Elektroden 114, 118 bilden dabei eine bipolare Elektrodenkonfiguration an dem Applikator 110 und sind an einem distalen Ende 119 des Applikators angeordnet. Dabei ist die erste Elektrode 114 an einer abgerundeten Spitze des distalen Endes 119 angeordnet und die zweite Elektrode 118 ist zwischen 0,5 cm und 3 cm von der ersten Elektrode 114 beabstandet.

Die Feedbackeinheit 130 ist mit der ersten und zweiten Elektrode 114, 118 elektrisch verbunden und ausgebildet, abhängig von einem elektrischen Widerstand zwischen der ersten und der zweiten Elektrode 114, 118 ein für einen Nutzer wahrnehmbares Ausgabesignal 134 zu generieren und auszugeben. Hierfür umfasst die Feedbackeinheit einen Tongenerator und einen Lautsprecher 138, so dass das Ausgabesignal 134 über den Lautsprecher 138 als für einen Nutzer des Elektrochirurgiesystems 100 hörbarer Ton ausgegeben werden kann. In dem dargestellten Ausführungsbeispiel ist der Tongenerator derart ausgebildet, dass die Frequenz des generierten Tons proportional zu dem elektrischen Widerstand zwischen der ersten Elektrode 114 und der zweiten Elektrode 118 ist. Für eine Behandlung von Körpergewebe steigt daher mit wachsendem elektrischem Widerstand des Gewebes, aufgrund einer durch eine Heizleistung der beiden Elektroden 114, 118 verursachten Austrocknung, die Tonhöhe des hörbaren Tons. Der Nutzer kann daher aus der Tonhöhe des Ausgabesignals 134 auf die Austrocknung des durch die beiden Elektroden 114, 118 behandelten Körpergewebes schließen.

Die Verarbeitungseinheit 140 ist mit der ersten und der zweiten Elektrode 114, 118 elektrisch verbunden. Sie ist ausgebildet ist, über die erste und die zweite Elektrode 114, 118 ein elektrisches Signal 145 aufzunehmen, daraus den elektrischen Widerstand oder einen den elektrischen Widerstand repräsentierenden Wert zwischen der ersten und der zweiten Elektrode 114, 118 zu bestimmen und abhängig von dem elektrischen Widerstand oder dem repräsentierenden Wert ein Steuersignal 148a, 148b zu generieren und auszugeben. Die Feedbackeinheit 130 und die Steuereinheit 150 sind außerdem ausgebildet, das Steuersignal 148a, 148b zu empfangen. Hierbei ist das Steuersignal als Feedback-Steuersignal 148a und als Steuereinheits-Steuersignal 148b ausgebildet, wobei das Feedback-Steuersignal 148a an die Feedbackeinheit 130 ausgegeben wird und das Steuereinheits-Steuersignal 148b an die Steuereinheit 150 ausgegeben wird. Diese beiden Steuersignale 148a, 148b unterscheiden sich nur aufgrund anderer Anschlüsse oder eines anderen Aufbaus von Feedbackeinheit 130 und Steuereinheit 150, jedoch nicht in Hinblick auf einen Informationsgehalt hinsichtlich des elektrischen Widerstands oder des den elektrischen Widerstand repräsentierenden Wertes.

Die Steuereinheit 150 ist ausgebildet, die Hochfrequenz-Spannungsversorgungseinheit 120 derart zu steuern, dass zwischen mindestens einem ersten und einem zweiten Betriebsmodus automatisch gewechselt werden kann, wobei im ersten Betriebsmodus die an den Elektroden 114, 118 anliegende Wechselspannung durch den Grenzwert U₁ und im zweiten Betriebsmodus durch den Grenzwert U₂ begrenzt ist. Hierbei ist U₁ größer als U₂. Weiterhin steuert die Steuereinheit 150 die Hochfrequenz-Spannungsversorgungseinheit 120 derart, dass ein automatischer Wechsel aus dem ersten Betriebsmodus in den zweiten Betriebsmodus dann erfolgt, wenn eine Anstiegsgeschwindigkeit des elektrischen Widerstands zwischen der ersten und zweiten Elektrode 114, 118 über einem vorbestimmten Grenzwert G liegt. Hierbei ist der zweite Grenzwert U₂ so gewählt, dass er zwischen 50% und 80%, bevorzugt 70% des ersten Grenzwertes U₁ beträgt. Die erste Grenzwert U₁ beträgt zwischen 80V und 200V, bevorzugt 100V. Die Steuerung durch die Steuereinheit 150 erfolgt in diesem Ausführungsbeispiel über ein entsprechendes Spannungssteuerungssignal 152 der Steuereinheit 150, dass von der Hochfrequenz-Spannungsversorgungseinheit 120 empfangen werden kann.

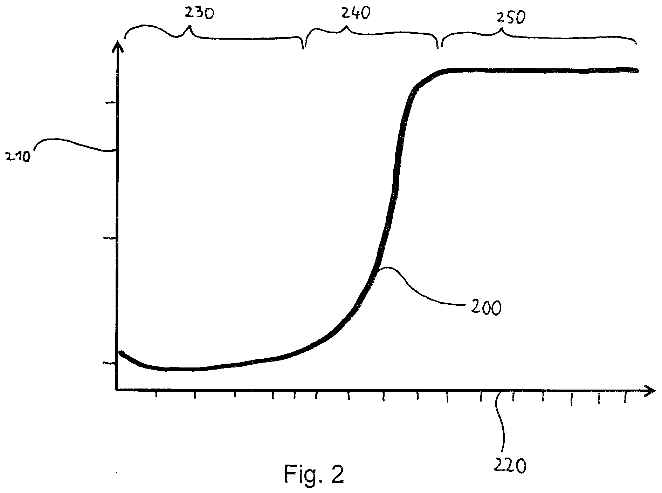

Ein Nutzer des Elektrochirurgiesystems 100 nutzt die Wechselspannung um dadurch das zu behandelnde Körpergewebe aufzuheizen und hört dabei durch die Tonhöhe des Ausgabesignals 134, wie groß der elektrische Widerstand zwischen den beiden Elektroden 114, 118 ist und dadurch auch, wie stark dieses Körpergewebe ausgetrocknet ist. Steigt der elektrische Widerstand im Rahmen einer Behandlung nahezu sprunghaft an, was dem in Fig. 2 veranschaulichten typischen Verlauf dieses elektrischen Widerstands entspricht, wird die Heizleistung der Elektroden 114, 118 durch ein Umschalten auf einen Grenzwert U₂ für die Wechselspannung reduziert. Dadurch wird die Anstiegsgeschwindigkeit des elektrischen Widerstands geringer wird und der Nutzer hat ausreichend Zeit, um auf das Ausgabesignal 134, beispielsweise durch eine Veränderung einer Position der beiden Elektroden 114, 118 an dem Körpergewebe, zu reagieren.

Weiterhin ist die Steuereinheit 150 ausgebildet, den Wert des elektrischen Widerstands zwischen der ersten Elektrode 114 und der zweiten Elektrode 118 oder einen den elektrischen Widerstand repräsentierenden Wert über einen vorbestimmten Zeitraum zu speichern. Dadurch kann die Anstiegsgeschwindigkeit des elektrischen Widerstands von der Steuereinheit 150 bestimmt werden, indem sie den elektrischen Widerstand oder den repräsentierenden Wert zu einem aktuellen Zeitpunkt mit dem elektrischen Widerstand oder dem repräsentierenden Wert zu einem früheren Zeitpunkt vergleicht. Ein Bestimmen der Anstiegsgeschwindigkeit erfolgt in diesem Fall durch Abschätzen dieser Anstiegsgeschwindigkeit mittels Differenzbildung.

Außerdem ist die Steuereinheit 150 ausgebildet, die Hochfrequenz-Spannungsversorgungseinheit 120 derart zu steuern, dass ein automatischer Wechsel aus dem zweiten Betriebsmodus in den ersten Betriebsmodus dann erfolgt, wenn der elektrische Widerstand zwischen der ersten und der zweiten Elektrode 114, 118 unter einem vorbestimmten Grenzwert W liegt. Hierbei ist der vorbestimmte Grenzwert W so gewählt, dass für den Fall, dass die beiden Elektroden 114, 118 an nicht ausgetrocknetem Gewebe anliegen, ein automatischer Wechsel in den ersten Betriebsmodus erfolgt. Hierdurch kann sichergestellt werden, dass der Nutzer des Elektrochirurgiesystems 100 sich nicht mit einem manuellen Umstellen der Heizleistung beschäftigen muss und sich somit besser auf die Positionierung der beiden Elektroden 114, 118 am zu behandelnden Körpergewebe konzentrieren kann.

In diesem gezeigten Ausführungsbeispiels des Elektrochirurgiesystems 100 ist der Applikator 110 so ausgebildet, dass er über einen Katheter zu dem zu behandelnden Körpergewebe zugeführt werden kann.

In einem nicht dargestellten Ausführungsbeispiel ist die Hochfrequenz-Spannungsversorgungseinheit mit einer ersten und zweiten Elektrode verbindbar, ohne jedoch mit den Elektroden oder dem Applikator verbunden zu sein. Die Elektroden können also zu einem externen Gerät gehören, dass kein Bestandteil des erfindungsgemäßen Elektrochirurgiesystems ist.

Das wahrnehmbare Ausgabesignal ist in nicht dargestellten Ausführungsbeispielen des Elektrochirurgiesystems als visuelles Signal ausgebildet, das ein Nutzer beispielsweise durch einen Monitor wahrnehmen kann.

Fig. 2 zeigt einen typischen Verlauf 200 des elektrischen Widerstands zwischen einer ersten und einer zweiten Elektrode über die Zeit bei der Thermonekrose von Körpergewebe.

Die Ordinate 210 zeigt den elektrischen Widerstand des behandelten Körpergewebes zwischen den zwei Elektroden in Ohm, wobei eine logarithmische Darstellung gewählt wurde, die von 10 Ohm am unteren Strich bis zu 1000 Ohm reicht. Auf der Abszisse 220 des den Verlauf 200 veranschaulichenden Diagramms ist die Zeit in 2 Sekunden Abschnitten aufgetragen.

Wie in durch den Verlauf 200 dargestellt, liegt ein typischer Gewebewiderstand zu Beginn einer Ablation von Körpergewebe bei ungefähr 10 Ohm. In einer Heizphase 230 zu Beginn der Ablation wird das Gewebe aufgeheizt, während der elektrische Widerstand ungefähr gleich bleibt. Danach erfolgt eine Sprungphase 240 in der der Gewebewiderstand nahezu sprunghaft ansteigt. In dieser Sprungphase 240 ist es für den Nutzer des Elektrochirurgiesystems schwer, rechtzeitig auf das Ausgabesignal zu reagieren, da der elektrische Widerstand innerhalb von nur 2 bis 5 Sekunden ansteigt. Nach diesem Sprung des elektrischen Widerstands ist das Gewebe in der Austrocknungsphase 250 und das Risiko besteht, dass Blut oder Gewebereste an den beiden mit dem Elektrochirurgiesystem verbundenen Elektroden anhaften und der Applikator gereinigt werden muss.

Im Rahmen einer wie gewünscht verlaufenden Ablation müsste der Nutzer während der Sprungphase 240 die Position der Elektroden so schnell verändern, dass die Elektroden rechtzeitig in die Nachbarschaft nicht ausgetrockneten Gewebes bewegt werden. Dieses hat zunächst wieder einen geringeren elektrischen Widerstand und wird durch die Elektroden aufgeheizt und eine neue Heizphase 230 beginnt. Wie im Rahmen von Fig. 1 beschrieben, kann die Steuereinheit des erfindungsgemäßen Elektrochirurgiesystems durch ein Reduzieren der Heizleistung der beiden Elektroden während der Sprungphase 240 die Dauer der Sprungphase 240 verlängern und somit eine Reaktion des Nutzers auf den sprunghaften Anstieg des elektrischen Widerstands des Gewebes erleichtern.

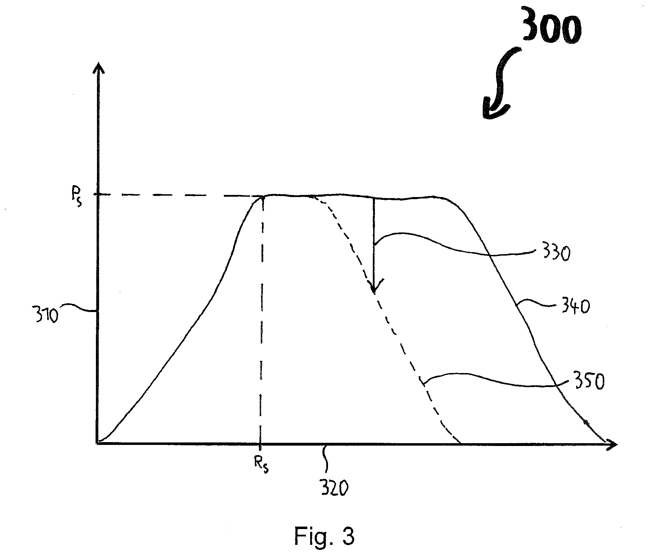

Fig. 3 zeigt einen Verlauf der Abhängigkeit 300 zwischen der von einer Hochfrequenz-Spannungsversorgungseinheit 120 an das Gewebe abgegebenen Heizleistung bei einer Ablation von Körpergewebe und einem elektrischen Widerstand, wobei ein Einfluss einer Reduktion 330 des Spannungsgrenzwertes von U₁ 340 auf U₂ 350 veranschaulicht wird.

Die Ordinate 310 zeigt die über die zwei Elektroden abgegebene Heizleistung in Watt. Auf der Abszisse 320 des die Abhängigkeit 300 veranschaulichenden Diagramms ist der elektrische Widerstand zwischen den beiden Elektroden in Ohm aufgetragen.

Die Abhängigkeit 300 zeigt, dass mit wachsendem elektrischem Widerstand des Körpergewebes bei kleinen Widerständen auch die Heizleistung steigt. In diesem Bereich ist wird die Heizleistung durch einen maximal zugelassenen Ausgangsstrom begrenzt. Die Heizleistung steigt dabei proportional mit dem Widerstand an. Sobald die Heizleistung auf einen voreingestellten Sollwert, z.B. 18 Watt, angestiegen ist erfolgt eine Leistungsbegrenzung, bei der Ausgangsstrom und Ausgangsspannung so geregelt werden, dass die Heizleistung dem Sollwert entspricht. Dabei sinkt der Ausgangsstrom mit steigendem Widerstand ab, während die Ausgangsspannung mit steigendem Widerstand zunimmt. In dem dargestellten Ausführungsbeispiel tritt die Leistungsbegrenzung bei einer Heizleistung Pₛ von 18 Watt und einem Widerstand Rₛ von ungefähr 200 Ohm ein. Bei weiter ansteigendem Widerstand wird die Heizleistung durch die maximal zugelassene Ausgangsspannung begrenzt und fällt umgekehrt proportional zum Widerstand ab.

Steigt nun die Anstiegsgeschwindigkeit des elektrischen Widerstandsüber einen Grenzwert G, was im dargestellten Ausführungsbeispiel bei 550 Ohm der Fall ist, erfolgt die Spannungsreduktion 330, durch die die maximal zulässige Ausgangsspannung, also der Grenzwert der Ausgangsspannung, von U₁ 340 mit einer Amplitude von 100V bis auf U₂ 350 mit einer Amplitude von 70V reduziert wird. Wie durch das Diagramm verdeutlicht, kann diese Reduktion 330 des Grenzwerts für eine Verringerung der Heizleistung um 50% sorgen.

Schaltet die Steuereinheit des Elektrochirurgiesystems von U₁ auf den Grenzwert U₂ mit der kleineren Amplitude, wird die über die beiden Elektroden abgegebene Heizleistung verringert, so dass der Nutzer gemäß dem Widerstandsverlauf nach Fig. 2 mehr Zeit hat, auf einen Anstieg des Gewebewiderstands zwischen der ersten und zweiten Elektrode zu reagieren.

Eine Spannungsreduktion aufgrund einer Anstiegsgeschwindigkeit des Widerstands über dem Grenzwert G erfolgt dabei typischerweise in einem Widerstandsbereich des zu behandelnden Gewebes zwischen 200 Ohm und 700 Ohm. Der Grenzwert G der Anstiegsgeschwindigkeit liegt dabei beispielsweise zwischen 10 und 100 Ohm/s, z.B. 50 Ohm/s.

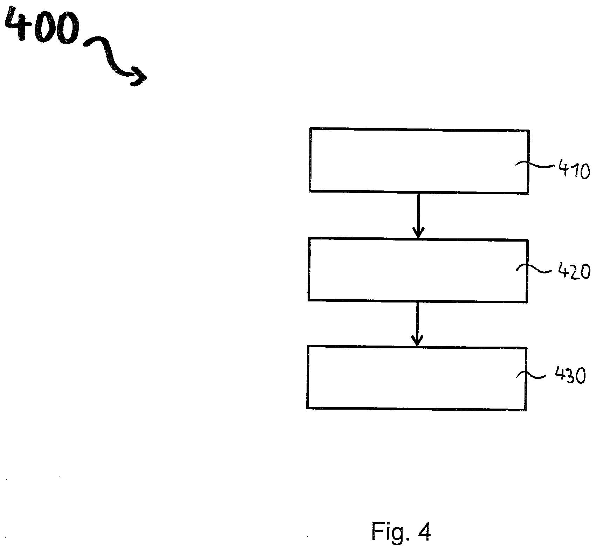

Fig. 4 zeigt ein nicht erfindungsgemäßes Verfahren 400 zum Betreiben eines Elektrochirurgiesystems gemäß einem weiteren Aspekt der Erfindung. Das Verfahren 400 umfasst drei Verfahrensschritte 410, 420, 430.

Ein erster Verfahrensschritt 410 besteht in einem Anlegen einer Wechselspannung an eine erste Elektrode und an eine zweite Elektrode eines Applikators.

In einem zweiten Verfahrensschritt 420 wird ein Ausgabesignal generiert und ausgegeben, wobei das Ausgabesignal von einem elektrischen Widerstand zwischen der ersten und der zweiten Elektrode abhängig ist.

Der dritte Verfahrensschritt 430 umfasst ein Steuern der Wechselspannung derart, dass zwischen mindestens einem ersten und einem zweiten Betriebsmodus des Applikators automatisiert gewechselt werden kann, wobei im ersten Betriebsmodus die Wechselspannung durch einen ersten Grenzwert U₁ und im zweiten Betriebsmodus durch eine zweiten Grenzwert U₂ begrenzt wird, wobei U₁ größer ist als U₂ hat und wobei ein automatisierter Wechsel aus dem ersten Betriebsmodus in den zweiten Betriebsmodus dann erfolgt, wenn eine Anstiegsgeschwindigkeit des elektrischen Widerstands zwischen der ersten und zweiten Elektrode über einem vorbestimmten Grenzwert G liegt.

### Bezuqszeichenliste:

- 100: Elektrochirurgiesystem
- 110: Applikator
- 114: erste Elektrode
- 118: zweite Elektrode
- 119: distales Ende
- 120: Hochfrequenz-Spannungsversorgungseinheit
- 130: Feedbackeinheit
- 134: Ausgabesignal
- 138: Lautsprecher
- 140: Verarbeitungseinheit
- 145: elektrisches Signal
- 148a: Feedback-Steuersignal
- 148b: Steuereinheits-Steuersignal
- 150: Steuereinheit
- 152: Spannungssteuerungssignal
- 155: Drehknopf
- 200: Verlauf des elektrischen Widerstands
- 210: Ordinate aus Fig. 2
- 220: Abszisse aus Fig. 2
- 230: Heizphase
- 240: Sprungphase
- 250: Austrocknungsphase
- 300: Abhängigkeit zwischen Heizleistung und Widerstand
- 310: Ordinate aus Fig. 3
- 320: Abszisse aus Fig. 3
- 330: Spannungsreduktion
- 340: Wechselspannung U₁
- 350: Wechselspannung U₂
- 400: erfindungsgemäßes Verfahren
- 410: erster Verfahrensschritt
- 420: zweiter Verfahrensschritt
- 430: dritter Verfahrensschritt

## Patentansprüche

1. Elektrochirurgiesystem (100) zum Generieren von hochfrequentem Wechselstrom für eine Ablation von Körpergewebe, mit
- einer Hochfrequenz-Spannungsversorgungseinheit (120), die mit einer ersten und zweiten Elektrode (114, 118) elektrisch verbunden oder zu verbinden ist und die ausgebildet ist, diese Elektroden mit einer hochfrequenten Wechselspannung zu versorgen;
- einer Feedbackeinheit (130), die mit der ersten und zweiten Elektrode (114, 118) elektrisch verbunden ist und die ausgebildet ist, ein von einem elektrischen Widerstand zwischen der ersten und der zweiten Elektrode abhängiges Ausgabesignal (134) zu generieren; und
- einer Steuereinheit (150), die ausgebildet ist, die Hochfrequenz-Spannungsversorgungseinheit (120) derart zu steuern, dass zwischen mindestens einem ersten und einem zweiten Betriebsmodus automatisiert gewechselt werden kann, wobei im ersten Betriebsmodus die Wechselspannung durch einen ersten Grenzwert U₁ und im zweiten Betriebsmodus die Wechselspannung durch einen zweiten Grenzwert U₂ begrenzt wird, wobei U₁ größer ist als U₂ und wobei ein automatisierter Wechsel aus dem ersten Betriebsmodus in den zweiten Betriebsmodus dann erfolgt, wenn eine Anstiegsgeschwindigkeit des elektrischen Widerstands zwischen der ersten und zweiten Elektrode (114, 118) über einem vorbestimmten Grenzwert G liegt,
**dadurch gekennzeichnet, dass**
die Steuereinheit (150) weiterhin ausgebildet ist, die Hochfrequenz-Spannungsversorgungseinheit (120) derart zu steuern, dass ein automatisierter Wechsel aus dem zweiten Betriebsmodus in den ersten Betriebsmodus dann erfolgt, wenn der elektrische Widerstand zwischen der ersten und der zweiten Elektrode (114, 118) unter einem vorbestimmten Grenzwert W liegt.

2. Elektrochirurgiesystem (100) gemäß Anspruch 1, weiterhin aufweisend eine Verarbeitungseinheit (140), die mit der ersten und der zweiten Elektrode (114, 118) elektrisch verbunden ist und ausgebildet ist, über die erste und die zweite Elektrode (114, 118) ein elektrisches Antwortsignal zu empfangen, daraus den elektrischen Widerstand oder einen den elektrischen Widerstand repräsentierenden Wert zwischen der ersten und der zweiten Elektrode (114, 118) zu bestimmen und abhängig von dem elektrischen Widerstand oder dem repräsentierenden Wert ein Steuersignal zu generieren und auszugeben, und wobei die Feedbackeinheit (130) und die Steuereinheit (150) weiterhin ausgebildet sind, das Steuersignal zu empfangen.

3. Elektrochirurgiesystem (100) gemäß einem der vorhergehenden Ansprüche, bei dem der zweite Grenzwert U₂ zwischen 50% und 80%, bevorzugt 70%, des ersten Grenzwerts U₁ beträgt.

4. Elektrochirurgiesystem (100) gemäß einem der vorhergehenden Ansprüche, bei dem die Steuereinheit (150) weiterhin ausgebildet ist, den elektrischen Widerstand zwischen der ersten Elektrode und der zweiten Elektrode (114, 118) oder einen den elektrischen Widerstand repräsentierenden Wert über einen vorbestimmten Zeitraum zu speichern, den elektrischen Widerstand oder den repräsentierenden Wert zu einem aktuellen Zeitpunkt mit dem elektrischen Widerstand oder dem repräsentierenden Wert zu einem früheren Zeitpunkt zu vergleichen und dadurch die Anstiegsgeschwindigkeit des elektrischen Widerstands zu bestimmen.

5. Elektrochirurgiesystem (100) gemäß einem der vorhergehenden Ansprüche, bei dem die Steuereinheit (150) weiterhin ausgebildet ist, zwischen einer Anzahl von weiteren Betriebsmodi mit weiteren Grenzwerten U₃,..., Uₙ der Wechselspannung mit der Reihe nach absteigenden Amplituden, die kleiner als die Amplitude von U₂ sind, automatisiert zu wechseln, wobei ein automatisierter Wechsel aus einem der Betriebsmodi Uᵢ in den Betriebsmodus Uᵢ₊₁ mit der nächst kleineren Amplitude der entsprechenden Wechselspannung dann erfolgt, wenn die Anstiegsgeschwindigkeit des elektrischen Widerstands zwischen der ersten und der zweiten Elektrode (114, 118) über einem vorbestimmten und dem Betriebsmodus Uᵢ zugeordneten Grenzwert liegt.

6. Elektrochirurgiesystem (100) gemäß einem der vorhergehenden Ansprüche, mit einem Applikator (110) bei dem die erste und die zweite Elektrode (114, 118) in dem Applikator (110) eine bipolare Elektrode bilden, wobei die erste Elektrode (114) an einer Spitze eines distalen Endes (119) des Applikators (110) angeordnet ist und die zweite Elektrode (118) zwischen 0,5 cm und 3 cm von der ersten Elektrode (114) beabstandet an dem distalen Ende (119) des Applikators (110) angeordnet ist.

7. Elektrochirurgiesystem (100) gemäß Anspruch 6, bei dem der Applikator (110) ausgebildet ist, über einen Katheter zu dem zu behandelnden Körpergewebe zuführbar zu sein.

## Claims

1. Electrosurgical system (100) for generating high-frequency alternating current for ablating body tissue, having
- a high-frequency voltage supply unit (120) which is connected or to be connected electrically to a first and second electrode (114, 118) and which is configured to supply these electrodes with a high-frequency alternating voltage;
- a feedback unit (130) which is electrically connected to the first and second electrodes (114, 118) and which is configured to generate an output signal (134) dependent on an electrical resistance between the first and the second electrode; and
- a control unit (150) which is configured to control the high-frequency voltage supply unit (120) such that it is possible to switch automatically between at least one first and one second operating mode, wherein in the first operating mode the alternating voltage is limited by a first threshold value U₁ and in the second operating mode the alternating voltage is limited by a second threshold value U₂, wherein U₁ is greater than U₂ and wherein an automated change from the first operating mode into the second operating mode takes place when a rate of increase in the electrical resistance between the first and second electrodes (114, 118) lies above a predetermined threshold value G,
**characterized in that**
the control unit (150) is furthermore configured to control the high-frequency voltage supply unit (120) such that an automated change from the second operating mode into the first operating mode takes place when the electrical resistance between the first and the second electrode (114, 118) is below a predetermined threshold value W.

2. Electrosurgical system (100) according to claim 1, furthermore having a processing unit (100) which is electrically connected to the first and the second electrodes (114, 118) and is configured to receive an electrical response signal via the first and the second electrodes (114, 118), to use this to determine the electrical resistance or a value representing the electrical resistance between the first and second electrodes (114, 118) and, depending on the electrical resistance or the representing value, to generate and output a control signal, and wherein the feedback unit (130) and the control unit (150) are furthermore configured to receive the control signal.

3. Electrosurgical system (100) according to any of the preceding claims, in which the second threshold value U₂ is between 50% and 80%, preferably 70%, of the first threshold value U₁.

4. Electrosurgical system (100) according to any of the preceding claims, in which the control unit (150) is furthermore configured to store the electrical resistance between the first electrode and the second electrode (114, 118), or a value representing the electrical resistance, during a predetermined period of time, to compare the electrical resistance or the representing value at a current point in time with the electrical resistance or the representing value at an earlier point in time and thereby to determine the rate of increase in the electrical resistance.

5. Electrosurgical system (100) according to any of the preceding claims, in which the control unit (150) is furthermore configured to change in an automated manner between a number of further operating modes with further threshold values U₃,..., Uₙ of the AC voltage with amplitudes decreasing sequentially which are smaller than the amplitude of U₂, wherein an automated change from one of the operating modes Uᵢ into the operating mode Uᵢ₊₁ with the next smaller amplitude of the appropriate AC voltage takes place when the rate of increase in the electrical resistance between the first and the second electrodes (114, 118) is above a threshold value which is predetermined and assigned to the operating mode Uᵢ.

6. Electrosurgical system (100) according to any of the preceding claims, having an applicator (110) in which the first and the second electrodes (114, 118) form a bipolar electrode in the applicator (110), wherein the first electrode (114) is arranged on a tip of a distal end (119) of the applicator (110) and the second electrode (118) is arranged on the distal end (119) of the applicator (110) at a distance of between 0.5 cm and 3 cm from the first electrode (114).

7. Electrosurgical system (100) according to claim 6, in which the applicator (110) is configured to be able to be supplied to the body tissue to be treated by way of a catheter.

## Revendications

1. Système électrochirurgical (100) pour générer un courant alternatif haute fréquence pour l'ablation de tissu corporel, avec
- une unité d'alimentation électrique haute fréquence (120), qui est raccordée ou doit être raccordée électriquement à une première et à une deuxième électrodes (114, 118) et qui est conçue pour alimenter ces électrodes avec une tension alternative haute fréquence,
- une unité de rétroaction (130), qui est raccordée électriquement à la première et à la deuxième électrodes (114, 118) et qui est conçue pour générer un signal de sortie (134) dépendant d'une résistance électrique entre la première et la deuxième électrodes ; et
- une unité de commande (150), qui est conçue pour commander l'unité d'alimentation électrique haute fréquence (120) de manière à pouvoir changer automatiquement entre au moins un premier et un deuxième mode de fonctionnement, dans lequel dans le premier mode de fonctionnement, la tension alternative est limitée par une première valeur limite U₁ et, dans le deuxième mode de fonctionnement, la tension alternative est limitée par une deuxième valeur limite U₂, dans lequel U₁ est supérieur à U₂ et dans lequel un changement automatique pour passer du premier mode de fonctionnement au deuxième mode de fonctionnement est effectué quand une vitesse d'augmentation de la résistance électrique entre la première et la deuxième électrodes (114, 118) se situe au-dessus d'une valeur limite G prédéfinie,
**caractérisé en ce que**
l'unité de commande (150) est conçue par ailleurs pour commander l'unité d'alimentation électrique haute fréquence (120) de telle manière qu'un changement automatique pour passer du deuxième mode de fonctionnement au premier mode de fonctionnement est effectué lorsque la résistance électrique entre la première et la deuxième électrodes (114, 118) se situe en dessous d'une valeur limite W prédéfinie.

2. Système électrochirurgical (100) selon la revendication 1, comportant par ailleurs une unité de traitement (140), qui est raccordée électriquement à la première et à la deuxième électrodes (114, 118) et est conçue pour recevoir un signal de réponse électrique par l'intermédiaire de la première et de la deuxième électrodes (114, 118), pour définir, sur cette base, la résistance électrique ou une valeur représentant la résistance électrique entre la première et la deuxième électrodes (114, 118) et pour générer et émettre en fonction de la résistance électrique ou de la valeur représentative un signal de commande, et dans lequel l'unité de rétroaction (130) et l'unité de commande (150) sont conçues par ailleurs pour recevoir le signal de commande.

3. Système électrochirurgical (100) selon l'une quelconque des revendications précédentes, où la deuxième valeur limite U₂ est comprise entre 50 % et 80 %, de manière préférée est de 70 %, de la première valeur limite U₁.

4. Système électrochirurgical (100) selon l'une quelconque des revendications précédentes, où l'unité de commande (150) est conçue par ailleurs pour mémoriser la résistance électrique entre la première électrode et la deuxième électrode (114, 118) ou une valeur représentant la résistance électrique sur une période de temps prédéfinie, pour comparer la résistance électrique ou la valeur représentative à un moment instantané à la résistance électrique ou à la valeur représentative à un moment antérieur et pour définir ainsi la vitesse d'augmentation de la résistance électrique.

5. Système électrochirurgical (100) selon l'une quelconque des revendications précédentes, où l'unité de commande (150) est conçue par ailleurs pour changer automatiquement entre un nombre donné d'autres modes de fonctionnement avec d'autres valeurs limites U₃, ..., Uₙ de la tension alternative avec des amplitudes baissant selon l'ordre, qui sont inférieures à l'amplitude de U₂, dans lequel un changement automatique d'un des modes de fonctionnement Uᵢ au mode de fonctionnement Uᵢ₊₁ avec l'amplitude immédiatement plus petite de la tension alternative correspondante est effectué lorsque la vitesse d'augmentation de la résistance électrique entre la première et la deuxième électrodes (114, 118) se situe au-dessus d'une valeur limite prédéfinie et associée au mode de fonctionnement Uᵢ.

6. Système électrochirurgical (100) selon l'une quelconque des revendications précédentes, avec un applicateur (110), où la première et la deuxième électrodes (114, 118) forment dans l'applicateur (110) une électrode bipolaire, dans lequel la première électrode (114) est disposée sur une pointe d'une extrémité distale (119) de l'applicateur (110) et la deuxième électrode (118) est disposée sur l'extrémité distale (119) de l'applicateur (110) à une distance entre 0,5 cm et 3 cm de la première électrode (114).

7. Système électrochirugical (100) selon la revendication 6, où l'applicateur (110) est réalisé pour pouvoir être amené au tissu corporel à traiter par l'intermédiaire d'un cathéter.
